# EUROPEAN PATENT SPECIFICATION

(11) **EP 2 134 251 B1**
(45) Date of publication and mention of the grant of the patent: **13.08.2014**
(21) Application number: 07752892.5
(22) Date of filing: 12.03.2007
(51) Int. Cl.: G01N 33/487, A61B 5/00, A61B 5/145, A61B 6/00

(54) **METHOD FOR USING A METER ADAPTED TO SIMULTANEOUSLY DISPLAY THE NUMBER OF HIGH, LOW AND TOTAL TEST RESULTS**
VERFAHREN ZUR VERWENDUNG EINES MESSGERÄTES ZUR GLEICHZEITIGEN ANZEIGE DER ANZAHL HOHER, NIEDRIGER UND GESAMT-TESTERGEBNISSE
PROCÉDÉ D'UTILISATION D'UN APPAREIL DE MESURE PERMETTANT D'AFFICHER SIMULTANÉMENT LES RÉSULTATS DE TESTS LES PLUS ÉLEVÉS, LES PLUS FAIBLES ET LES TOTAUX

(43) Date of publication of application: 23.12.2009
(73) Proprietor: Bayer HealthCare LLC, Whippany, NJ 07981-0915 (US)
(72) Inventor: POWER, Barry, D., Elkhart, IN 46514 (US); CULVER, Jeffrey, A., Sylvania, OH 43560-9655 (US)
(74) Representative: Linhart, Angela
(86) International application number: PCT/US2007/006224
(87) International publication number: WO 2008/111935

(56) References cited:
- WO-A-03/065033
- WO-A-2005/065538
- WO-A-2006/017358
- WO-A-2006/133348

## Description

The present invention relates generally to testing analyte concentrations and more particularly to methods of using the a meter adapted to simultaneously display the number of test results associated with analyze concentration readings above a predetermined analyte concentration level, the number of test results associated with analyte concentration, readings below a predetermined analyte concentration level and the total number of readings taken during a predetermined time interval.

The quantitative determination of analytes in body fluids is of great importance in the diagnoses and maintenance of certain physiological abnormalities. For example, lactate, cholesterol, bilirubin and glucose should be monitored in certain individuals. In particular, determining the concentration of glucose is important to diabetic individuals who must frequently check the glucose concentration in their body fluids to regulate the glucose intake in their diets. A person can become very ill if the blood glucose level becomes too high - a condition known as hyperglycemia. Consequently, for some individuals, it is important to be able to determine how often the glucose concentration readings exceed a certain glucose concentration level. When blood glucose levels drop too low - a condition known as hypoglycemia - a person can become nervous, shaky, and confused. That person's judgment may become impaired and that person may eventually pass out. Thus, it is also important for some individuals to be able to determine how often the glucose concentration readings fall below a certain glucose concentration level.

Diabetic individuals often test their blood glucose levels via a blood glucose meter. Some current meters allow users to save the test results. However, such meters do not allow users to review the test results in a manner that is most useful to the user and to healthcare professionals. Healthcare professionals recognize that it is important for some diabetic individuals to quickly ascertain glucose test results, particularly those test results associated with high and low concentration levels. This allows the healthcare professional to determine the individual's blood glucose testing compliance and progress of the individual's disease state.
For analysing a plurality of blood glucose readings taken in a predetermined time period it is known (WO 03/065033) to use a computer system for calculation purposes but the blood glucose readings have to taken before by a separate device.

To that end, it would be desirable to have a meter that displays testing results in a manner that would be useful to the user and/or healthcare professional and that would provide the user and/or healthcare professional with a quick summary of test results so that the healthcare professional can better evaluate and adjust an individual's diabetes care therapies.

The object of the present invention is to enable to provide users like healthcare professionals with testing results of analyte concentrations which are useful for better evaluating and adjusting individual diabetis care therapies.

This object is achieved by a method comprising the features of claim 1.
Further features and improvements of this method are subject matter of the claims dependent to claim 1.

A meter is disclosed which is adapted to determine an analyte concentration. The meter comprises a display adapted to display information to a user of the meter comprising a first value representing the number of analyte concentration readings that are greater than a first predetermined analyte concentration level, a second value representing the number of analyte concentration readings that are less than a second predetermined analyte concentration level and a third value representing the total number of analyte concentration readings taken over a predetermined time interval. The first value, the second value and the third value are simultaneously displayed to a user of the meter.

A method for using a meter adapted to determine an analyte concentration is disclosed according to one embodiment of the present invention. The meter has a display adapted to display information to a user. The method includes the acts of simultaneously displaying a first value, a second value and a third value. The first value represents the number of analyte concentration readings that are greater than a first predetermined analyte concentration level. The second value represents the number of analyte concentration readings that are less than a second predetermined analyte concentration level. The third value represents the total number of analyte concentration readings taken over a predetermined time interval.

### BRIEF DESCRIPTION OF THE DRAWING

FIG. 1 is a front view of a meter for using the present invention having a display for displaying the number of concentration readings pertaining to high concentration readings, low concentration readings and the total number of concentration readings in a given time period.
FIG. 2 is a front view of a meter of another embodiment having a display for displaying the number of concentration readings pertaining to high concentration readings, low concentration readings and the total number of concentration readings in a given time period.

The present invention uses a meter that is adapted to determine an analyte concentration in a body fluid sample which is collected with a lancing device. Examples of the types of analytes which may be collected include glucose, lipid profiles (e.g., cholesterol, triglycerides, LDL and HDL), microalbumin, hemoglobin A_{IC}, fiuctose, lactate, or bilirubin. It is contemplated that other analyte concentrations may also be determined. The analytes may be in, for example, a whole blood sample, a blood serum sample, a blood plasma sample, other body fluids like ISF (interstitial fluid) and urine, and non-body fluids. As used within this application, the term "concentration" refers to an analyte concentration, activity (e.g., enzymes and electrolytes), titers (e.g., antibodies), or any other measure concentration used to measure the desired analyte.

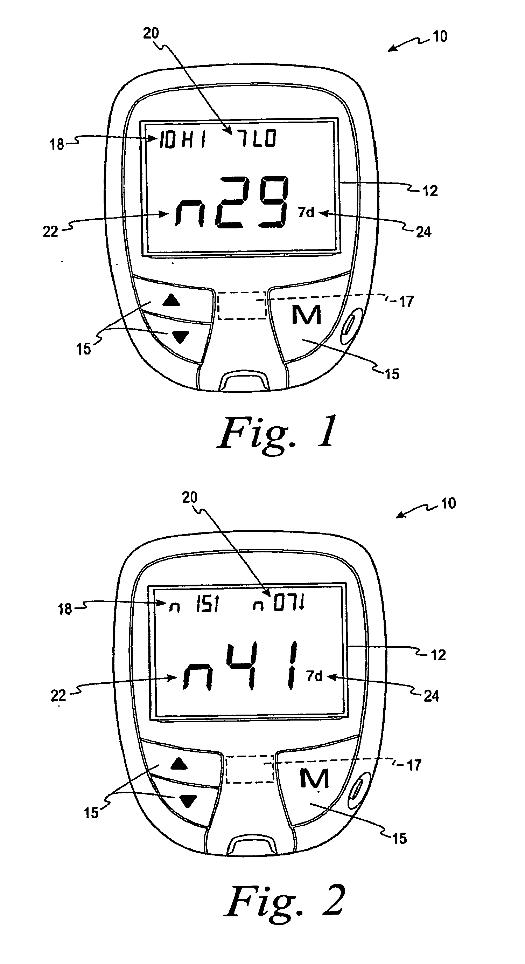

One embodiment of the meter 10 is shown in FIG. 1. The meter 10 has a display 12 that is adapted to display information to a user of the meter 10. Some of the information that may be displayed to a user includes concentration readings, time and date indicators, markers, alarms and any combination of such items. The meter 10 also has at least one user input mechanism 15 that is adapted to allow the user to make selections relating to one or more user features. The user input mechanism 15 may include, for example, buttons, scroll bars, touch screens, or any combination of such items. The meter 10 in this embodiment also has a memory device 17 that is adapted to store concentration readings, etc.

The display 12 may include any of several types of displays. For example, the display 12 may include an LCD display, a graphics display, a plasma display, a backlit display, a combination segmented/graphic display or any other suitable display. It is contemplated that the embodiments of the present invention may be displayed on other meters that are adapted to determine analyze concentrations.

According to one embodiment, the meter 10 includes a feature for simultaneously displaying testing results associated with high and low analyte concentration readings that have been taken by the user and stored in the meter 10. In one portion of the display 12, the meter 10 evaluates and displays the total number of analyte concentration readings that are greater than a predetermined analyte concentration level. For example, in FIG. 1, the first value 18 in the upper left hand corner, i.e., "10," corresponds to the number of concentration readings that exceed a predetermined analyte concentration level. In some embodiments, the first value 18 may be preceded or followed by letters, symbols, characters, etc. to indicate that this value represents the number of high analyte concentration readings. For example, in FIG. 1, following the first value 18 are the letters "HI" to indicate that the first value 18 represents the number of high analyte concentration readings. Similarly, in FIG. 2, the first value 18 in the upper left hand corner, i.e., "15," is followed by a symbol, i.e., "↑" to also indicate that the first value 18 represents the number of high analyte concentration readings.

In another portion of the display 12, the meter 10 evaluates and displays the total number of analyte concentration readings that are less than a predetermined analyte concentration level. For example, in FIG. 1, the second value 20 in the upper right hand corner, i.e., "7," corresponds to the number of concentration readings that falls below a predetermine analyte concentration level. In some embodiments, the second value 20 may be preceded or followed by letters, symbols, characters, etc. to indicate that this value represents the number of low analyte concentration readings i.e., "LO" (FIG. 1) or "↓" (FIG. 2). The order and location of the first value 18 and the second value 20 on the display 12 may vary as long as the first value 18 and the second value 20 are displayed to a user simultaneously.

The predetermined analyte concentration levels may be pre-programmed via default values that are set prior to an individual's use of the meter 10. Alternatively, values may be pre-programmed such that a user can select from a list of values pre-set by the meter 10. Additionally, the predetermined analyte concentration levels may be programmed or inputted by the user via the user input mechanism 15. The pre-programmed or programmable analyte concentration levels may be established when the meter 10 is initially in "set-up" mode. For example, a user who is testing for glucose may select or input 180 mg/dL or 10 mmol/L as a first predetermined analyte concentration level (corresponding to a "high" reading). The user may also select or input 72 mg/dL or 4.0 mmol/L as a second predetermined analyte concentration level (corresponding to a "low" reading) for glucose. These levels may be changed at various points throughout an individual's testing regime. For example, a user may enter "set-up" mode at a later time, after initially establishing the first and second analyte concentration levels, to change one or both of the analyte concentration levels to correspond to the needs of the patient's or as the testing regime is modified.

The unit of measure for these levels may be preprogrammed for particular concentration units, such as, for example, mg/dL or mmol/L. The particular unit of measure used may depend certain factors, such as the geographic market of use convention. For example, in the U.S., the preferred concentration units for a particular analyte may include "mg/dL;" while in Europe, the preferred concentration units may include "mmol/L."

In addition to displaying the first value 18 (indicating the number of high concentration readings) and the second value 20 (indicating the number of low concentration readings), the meter 10 may also display a third value 22 representing the total number of analyte concentration readings taken over a predetermined time interval, as shown in FIGS. 1 and 2 as "29" and "41" respectively. Also shown in FIGS. 1 and 2 is a time-interval indicator 24 that indicates the time interval over which the analyte concentration readings were evaluated. In the embodiments shown in FIGS. 1 and 2, the time interval indicator 24 displays **7d,* i.e., 7 days. As with the predetermined analyte concentration levels described above, the predetermined time interval may be pre-programmed, via a default time interval value or by a user selecting from a list of values pre-set by the meter 10, or may be programmed or inputted by the user via the user input mechanism 15. The pre-programmed or programmable time interval may be established when the meter 10 is initially in set-up mode. Other time intervals that may be pre-programmed include, for example, 14 days, 21 days, 1 month, 2 months, 3 months, etc.

Thus, the embodiments described above provide meters that calculate, retain and display analyte concentration readings in an aggregate and meaningful way. Providing this information in a single display quickly allows for a determination of the amount of variability in analyte concentration readings and assists the user and the healthcare professional in the treatment of the user's disease or condition. For example, after reviewing the number of high and low glucose concentration readings against the total number of tests conducted over a predetermined time period, the diabetic individual and/or the healthcare professional will be able to quickly determine if the individual is achieving tight glycemic control, which refers to maintaining blood glucose levels that do not significantly vary over a given time period. For example, if the number of high and low analyte concentration readings in a given time interval is small, then the user is achieving tight glycemic control. If the number of high and low analyte concentration readings in a given time interval is high, tight glycemic control is not being achieved and the healthcare professional can recommend corrective action through further laboratory testing, personal regimen change and clinical therapies to avoid long-term complications associated with the disease.

To illustrate the use of the meter 10 and the embodiments described herein, once a blood glucose concentration reading is calculated, this value is saved via the memory device 17. After obtaining and saving additional readings for a certain time period, the user (or healthcare professional) may select an option of the meter 10 for evaluating all analyte concentration readings taken during a specified time interval. The meter compares all readings that have been saved during the specified time interval with the first predetermined analyte concentration level (for "high" readings) and the second predetermined analyte concentration level (for "low" readings)..The meter then determines the number of readings that exceed the "high" level and the number of readings that fall below the "low" level and displays those values 18, 20 representing the number of readings on the display 12. The total number of readings 22 taken during the time interval is also displayed.

It is also contemplated that an alternative embodiment may include a meter 10 that displays only the first value 18 associated with the number of high concentration analyte readings and the second value 20 associated with the number of low concentration analyte readings on the display 12. Such information may alert the user and/or the healthcare professional that additional testing and/or corrective action should be considered.

Some commercially available meters, such as those that are manufactured and/or sold by Bayer Healthcare LLC of Tarrytown, New York, may be designed to incorporate embodiments of the present invention, such as the Ascensia® CONTOUR® Blood Glucose Monitoring System, the Ascensia® BREEZE® Blood Glucose Monitoring System and the Ascensia® Elite® and Elite® XL Blood Glucose Monitoring System. It is contemplated that other meters, in addition to the ones listed above, may incorporate embodiments of the present invention as described herein.

The features of the meter 10 described herein may be displayed in other formats, i.e., using different numeric characters, symbols, words, etc., in addition to the ones described herein. For example, instead of "HI" and "LO", the meter could display "HIGH" or "LOW" or, alternatively, "HYPO" or "HYPER." Additionally, the locations of the values displayed by the meter 10 may occur on different parts of the display 12 and may or may not occur in combination with other characters, symbols, text, etc. It is also contemplated that other values, indicators, markers and features may be displayed on the display 12 in addition to the features described herein. Also, while the embodiments described herein include references to glucose testing, it is contemplated that such embodiments could be used for testing other analytes in a fluid sample in addition to glucose.

### ALTERNATIVE EMBODIMENT A

A meter adapted to determine an analyte concentration, the meter comprising a display adapted to display information to a user of the meter comprising:
a first value representing the number of analyte concentration readings that are greater than a first predetermined analyte concentration level;
a second value representing the number of analyte concentration readings that are less than a second predetermined analyte concentration level; and
a third value representing the total number of analyte concentration readings taken over a predetermined time interval, wherein the first value, the second value and the third value are simultaneously displayed to a user of the meter.

### ALTERNATIVE EMBODIMENT B

The meter according to alternative embodiment A, wherein the display comprises an LCD display, a graphics display, a plasma display, a backlit display, or a combination segmented/graphic display.

### ALTERNATIVE EMBODIMENT C

The meter according to alternative embodiment A, wherein the analyte concentration reading is a glucose concentration reading.

### ALTERNATIVE EMBODIMENT D

The meter according to alternative embodiment A, wherein at least one of the first predetermined analyte concentration level and the second predetermined analyte concentration level is pre-programmed.

### ALTERNATIVE EMBODIMENT E

The meter according to alternative embodiment A, wherein at least one of the first predetermined analyte concentration level and the second predetermined analyte concentration level is input by the user via a user input mechanism.

### ALTERNATIVE EMBODIMENT F

The meter according to alternative embodiment A, wherein the predetermined time interval is pre-programmed.

### ALTERNATIVE EMBODIMENT G

The meter according to alternative embodiment A, wherein the predetermined time interval is input by the user via a user input mechanism.

### ALTERNATIVE PROCESS H

A method for using a meter adapted to determine an analyte concentration, the meter having a display adapted to display information to a user, the method comprising the act of simultaneously displaying a first value, a second value and a third value, the first value representing the number of analyte concentration readings that are greater than a first predetermined analyte concentration level, the second value representing the number of analyte concentration readings that are less than a second predetermined analyte concentration level and the third value representing the total number of analyte concentration readings taken over a predetermined time interval.

### ALTERNATIVE PROCESS I

The method according to alternative process H, herein, the display for simultaneously displaying the first value, the second value and the third value includes an LCD display, a graphics display, a plasma display, a backlit display, or a combination segmented/graphic display.

### ALTERNATIVE PROCESS J

The method according to alternative process H, wherein the analyte concentration reading is a glucose concentration reading.

### ALTERNATIVE PROCESS K

The method according to alternative process H, the method further comprising evaluating the first value, the second value and the third value to determine if tight glycemic control is achieved.

### ALTERNATIVE PROCESS L

The method according to alternative process H, further comprising allowing the user to input at least one of the predetermined time interval, the first predetermined analyte concentration level and the second predetermined analyte concentration level via a user input mechanism.

### ALTERNATIVE PROCESS M

The method according to alternative process H, further comprising allowing the user to select the predetermined time interval from a plurality of pre-programmed time intervals.

### ALTERNATIVE PROCESS N

The method according to alternative process H, further comprising allowing the user to select at least one of the first predetermined analyte concentration level and the second predetermined analyte concentration level from a plurality of pre-programmed analyte concentration levels.

## Claims

1. A method for using a meter adapted to determine an analyte concentration, the meter (10) having a display (12) adapted to display information to a user, the method comprising determining, via the meter, analyte concentration readings from fluid samples, storing the analyte concentration readings in a memory (17) of the meter; and simultaneously displaying on the meter a first value (18), a second value (20) and a third value (22), the first value representing the number of analyte concentration readings that are greater than a first predetermined analyte concentration level, the second value representing the number of analyte concentration readings that are less than a second predetermined analyte concentration level and the third value representing the total number of analyte concentration readings taken over a predetermined time interval wherein the first and the second predetermined analyte concentration levels are different.

2. The method according to claim 1, **characterized in that** the display for simultaneously displaying the first value, the second value and the third value includes an LCD display, a graphics display, a plasma display, a backlit display, or a combination segmented/graphic display.

3. The method according to claim 1, **characterized in that** the analyte concentration reading is a glucose concentration reading.

4. The method according to claim 1, **characterized in that** the method further comprises evaluating the first value, the second value and the third value to determine if tight glycemic control is achieved.

5. The method according to claim 1, **characterized in that** the user is allowed to input at least one of the predetermined time interval, the first predetermined analyte concentration level and the second predetermined analyte concentration level via a user input mechanism (15).

6. The method according to claim 1, **characterized in that** the user is allowed to select the predetermined time interval from a plurality of pre-programmed time intervals.

7. The method according to claim 1, **characterized in that** the user is allowed to select at least one of the first predetermined analyte concentration level and the second predetermined analyte concentration level from a plurality of pre-programmed analyte concentration levels.

## Patentansprüche

1. Verfahren zur Verwendung eines Messgerätes zur Bestimmung einer Analytkonzentration, wobei das Messgerät (10) ein Display (12) zum Anzeigen von Informationen für einen Anwender aufweist, wobei das Verfahren die Bestimmung über das Messgerät von Analytkonzentrationsmessungen von Flüssigkeitsproben, das Speichern der Analytkonzentrationsmessungen in einem Speicher (17) des Messgeräts; und die gleichzeitige Anzeige auf dem Messgerät eines ersten Werts (18), eines zweiten Werts (20) und eines dritten Werts (22) umfasst, wobei der erste Wert die Anzahl der Analytkonzentrationsmessungen darstellt, die größer als ein erstes vorbestimmtes Analytkonzentrationsniveau ist, der zweite Wert die Anzahl der Analytkonzentrationsmessungen darstellt, die kleiner als ein zweites vorbestimmtes Analytkonzentrationsniveau ist, und der dritte Wert die Gesamtzahl der Analytkonzentrationsmessungen darstellt, die während einer vorbestimmten Zeitspanne durchgeführt wurden, worin die ersten und zweiten vorbestimmten Analytkonzentrationsniveaus unterschiedlich sind.

2. Verfahren nach Anspruch 1, **dadurch gekennzeichnet, dass** das Display zur gleichzeitigen Anzeige des ersten Werts, des zweiten Werts und des dritten Werts ein LCD-Display, ein grafisches Display, ein Plasmadisplay, ein von hinten beleuchtetes Display oder eine Kombination aus segmentiertem/grafischen Display umfasst.

3. Verfahren nach Anspruch 1, **dadurch gekennzeichnet, dass** die Analytkonzentrationsmessung eine Blutzuckerkonzentrationsmessung ist.

4. Verfahren nach Anspruch 1, **dadurch gekennzeichnet, dass** das Verfahren ferner die Auswertung des ersten Werts, des zweiten Werts und des dritten Werts zur Bestimmung, ob eine enge glykämische Kontrolle erreicht wurde, umfasst.

5. Verfahren nach Anspruch 1, **dadurch gekennzeichnet, dass** der Anwender zumindest die vorbestimmte Zeitspanne, das erste vorbestimmte Analytkonzentrationsniveau und/oder das zweite vorbestimmte Analytkonzentrationsniveau über einen Anwendereingabemechanismus (15) eingeben darf.

6. Verfahren nach Anspruch 1, **dadurch gekennzeichnet, dass** der Anwender die vorbestimmte Zeitspanne aus einer Vielzahl von vorprogrammierten Zeitspannen auswählen darf.

7. Verfahren nach Anspruch 1, **dadurch gekennzeichnet, dass** der Anwender zumindest das erste vorbestimmte Analytkonzentrationsniveau und/oder das zweite vorbestimmte Analytkonzentrationsniveau aus einer Vielzahl von vorprogrammierten Analytkonzentrationsniveaus auswählen darf.

## Revendications

1. Procédé d'utilisation d'un instrument de mesure prévu pour déterminer une concentration d'analyte, l'instrument (10) de mesure comprenant un affichage (12) prévu pour présenter des informations à un utilisateur, le procédé comportant les étapes consistant à déterminer, via l'instrument de mesure, des indications de concentration d'analyte à partir d'échantillons de fluide, à conserver les indications de concentration d'analyte dans une mémoire (17) de l'instrument de mesure ; et à afficher simultanément sur l'instrument de mesure une première valeur (18), une deuxième valeur (20) et une troisième valeur (22), la première valeur représentant le nombre d'indications de concentration d'analyte qui sont supérieures à un premier niveau prédéterminé de concentration d'analyte, la deuxième valeur représentant le nombre d'indications de concentration d'analyte qui sont inférieures à un deuxième niveau prédéterminé de concentration d'analyte et la troisième valeur représentant le nombre total d'indications de concentration d'analyte relevées sur un intervalle de temps prédéterminé, le premier et le deuxième niveau prédéterminé de concentration d'analyte étant différents.

2. Procédé selon la revendication 1, **caractérisé en ce que** l'affichage servant à afficher simultanément la première valeur, la deuxième valeur et la troisième valeur comprend un affichage LCD, un affichage graphique, un affichage à plasma, un affichage rétro-éclairé ou un affichage segmenté / graphique combiné.

3. Procédé selon la revendication 1, **caractérisé en ce que** l'indication de concentration d'analyte est une indication de concentration de glucose.

4. Procédé selon la revendication 1, **caractérisé en ce que** le procédé comporte en outre l'étape consistant à évaluer la première valeur, la deuxième valeur et la troisième valeur pour déterminer si une régulation glycémique stricte est réalisée.

5. Procédé selon la revendication 1, **caractérisé en ce que** l'utilisateur est habilité à saisir au moins un paramètre parmi l'intervalle de temps prédéterminé, le premier niveau prédéterminé de concentration d'analyte et le deuxième niveau prédéterminé de concentration d'analyte via un mécanisme (15) de saisie d'utilisateur.

6. Procédé selon la revendication 1, **caractérisé en ce que** l'utilisateur est habilité à sélectionner l'intervalle de temps prédéterminé parmi une pluralité d'intervalles de temps préprogrammés.

7. Procédé selon la revendication 1, **caractérisé en ce que** l'utilisateur est habilité à sélectionner au moins un paramètre parmi le premier niveau prédéterminé de concentration d'analyte et le deuxième niveau prédéterminé de concentration d'analyte parmi une pluralité de niveaux préprogrammés de concentration d'analyte.
